**Europäisches Patentamt**

**European Patent Office**

**Office européen des brevets**

(19)

(11) Veröffentlichungsnummer: **0 105 474**
**B1**

(12) **EUROPÄISCHE PATENTSCHRIFT**

(45) Veröffentlichungstag der Patentschrift:
**23.10.85**

(21) Anmeldenummer: **83109741.5**

(22) Anmeldetag: **29.09.83**

(51) Int. Cl.⁴: **C 07 C 49/403,** C 07 C 45/83,
C 07 C 45/81 // C07C45/45

(54) Verfahren zur Isolierung von kristallinem 1,3-Cyclohexandion.

(30) Priorität: **04.10.82 DE 3236701**
**04.10.82 DE 3236700**

(43) Veröffentlichungstag der Anmeldung:
**18.04.84 Patentblatt 84/16**

(45) Bekanntmachung des Hinweises auf die Patenterteilung:
**23.10.85 Patentblatt 85/43**

(84) Benannte Vertragsstaaten:
**BE CH DE FR GB IT LI NL**

(56) Entgegenhaltungen:
**DE - A - 2 412 313**
**US - A - 4 399 310**

(73) Patentinhaber: **HOECHST AKTIENGESELLSCHAFT,**
**Postfach 80 03 20, D-6230 Frankfurt am Main 80 (DE)**

(72) Erfinder: **Litterer, Heinz, Dr., Albert-Schweitzer-Allee 39,**
**D-6200 Wiesbaden (DE)**
Erfinder: **Meidert, Helmut, Dr., Griesheimer Stadtweg 11,**
**D-6230 Frankfurt am Main 80 (DE)**

## Beschreibung

Bei der dehydratisierenden Cyclisierung von 5-Oxohexansäure zu 1,3-Cyclohexandion an Festbettkatalysatoren in der Gasphase erhält man nach den bekannten Verfahren (z. B. nach DE-OS 2 448 677) ein Produktgemisch — im folgenden meist als »Rohprodukt« bezeichnet — das neben dem gewünschten 1,3-Cyclohexandion hauptsächlich 6-Methyl-3,4-dihydro-2-pyranon, unumgesetzte 5-Oxohexansäure und das Reaktionswasser enthält.

Die Isolierung von reinem, kristallinem 1,3-Cyclohexandion aus diesem Rohprodukt ist mit außerordentlichen Schwierigkeiten verbunden. Das 1,3-Cyclohexandion liegt nämlich nach Kondensation des Rohprodukts zunächst ganz oder weitgehend in gelöster Form vor. Das Rohprodukt kristallisiert zwar beim Stehenlassen in zunehmendem Maße, dabei entsteht aber ein Kristallbrei von honigähnlicher Konsistenz, der in technisch akzeptablen Zeiträumen praktisch nicht filtrierbar ist. Das im Rohprodukt enthaltene 1,3-Cyclohexandion kristallisiert zudem nur sehr unvollständig und in stark verunreinigter Form aus.

Setzt man die Viskosität des erwähnten Kristallbreis durch Zusatz eines Verdünnungsmittels herab, so stellt man fest, daß das abfiltrierte kristalline 1,3-Cyclohexandion mit wesentlichen Mengen von kristallinem 5-Oxohexansäure-Hydrat verunreinigt ist.

Angesichts der geschilderten Schwierigkeiten überrascht es nicht, daß in der Literatur die Menge des bei der Cyclisierung von 5-Oxohexansäure entstandenen 1,3-Cyclohexandions nur gaschromatographisch bestimmt wurde, während ein technisch gangbarer Weg zur Reinisolierung von kristallinem 1,3-Cyclohexandion aus dem Rohprodukt bisher nicht beschrieben wurde.

Die durch die vorliegende Erfindung gelöste Aufgabe besteht darin, reines, kristallines 1,3-Cyclohexandion in hoher Ausbeute und mit genügend großer Filtrationsgeschwindigkeit zu isolieren.

Ein Gegenstand der vorliegenden Erfindung ist ein Verfahren zur Isolierung von kristallinem 1,3-Cyclohexandion aus dem bei der katalytischen Gasphase-Cyclisierung von 5-Oxohexansäure gebildeten Rohprodukt, das dadurch gekennzeichnet ist, daß man dem Rohprodukt vor oder direkt nach der Kondensation 20 bis 200 Gew.-%, bezogen auf das Rohprodukt, einer Hilfskomponente zugibt, die aus mindestens einer Verbindung aus der von cyclischen Kohlenwasserstoffen, chlorierten aliphatischen Kohlenwasserstoffen und Monoethern gebildeten Gruppen besteht, dann die Mischung aus Rohprodukt und Hilfskomponente so lange unter normalem oder vermindertem Druck bei 0 bis 80°C destilliert, bis das Reaktionswasser entfernt ist und der Gehalt an Hilfskomponente im zurückbleibenden Konzentrat 5 bis 50 Gew.-% beträgt, und das danach ausgefallene kristalline 1,3-Cyclohexandion abfiltriert. Vorzugsweise destilliert man bei 10 bis 40°C. Dabei sind die soeben und im folgenden angegebenen Destillationstemperaturen stets die im Sumpf gemessenen Temperaturen.

Die als Hilfskomponente in Frage kommenden cyclischen Kohlenwasserstoffe können Aromaten oder Cycloaliphaten, sowie chloriert oder unchloriert sein. Sie haben im allgemeinen bis zu 12 C-Atome. Bevorzugt sind Benzol, Toluol, Xylol, Mesitylen, Cumol, Ethylbenzol, Chlorbenzol, Dichlorbenzol, Tetralin, Dekalin, Cyclohexan und Methylcyclohexan. Besonders bevorzugt sind Xylol, Mesitylen, Cumol, Ethylbenzol, Chlorbenzol, Dichlorbenzol, Tetralin und Dekalin.

Die chlorierten aliphatischen Kohlenwasserstoffe haben im allgemeinen bis zu 6 C-Atome. Bevorzugt sind Methylenchlorid, Chloroform, Tetrachlorkohlenstoff, Dichlorethan, Tetrachlorethan und Butylchlorid. Besonders bevorzugt ist Tetrachlorethan.

Die Monoether können aliphatisch oder aromatisch sein. Sie haben im allgemeinen bis zu 12 C-Atome; bevorzugt sind Diisopropylether, Di-n-butylether und Di-n-hexylether, Anisol und Phenetol; besonders bevorzugt ist Di-n-butylether.

Die am besten geeigneten Hilfskomponenten sind Xylol und Di-n-butylether.

Die Hilfskomponente wird in Mengen von 20 bis 200 Gew.-%, vorzugsweise 20 bis 100 Gew.-%, insbesondere 20 bis 80 Gew.-%, bezogen auf das Rohprodukt, zugesetzt, wobei die optimale prozentuale Menge sich innerhalb dieser Grenzen nach dem Wassergehalt des Rohprodukts und nach dem Wassergehalt des Azeotrops aus Wasser und jeweiliger Hilfskomponente richtet. Zum Beispiel enthält ein Wasser-Xylol-Azeotrop unter sonst gleichen Bedingungen ca. 10mal soviel Wasser wie ein Wasser-Diisopropylether-Azeotrop.

Es ist vorteilhaft, den bei der Cyclisierung von 5-Oxohexansäure gebildeten, gasförmig aus dem Reaktor austretenden Produktstrom unmittelbar nach dessen Kondensation mit der Hilfskomponente zu vermischen bzw. den gasförmigen Produktstrom direkt in der vorgelegten Hilfskomponente, vorzugsweise bei Temperaturen bis zu 20°C, zu kondensieren. In einer besonders vorteilhaften Ausführungsform des erfindungsgemäßen Verfahrens werden gasförmiger Produktstrom und flüssige Hilfskomponente am Reaktorausgang zusammengeführt, bevor diese Mischung dann vollständig kondensiert wird. Dies ist von besonderer Bedeutung für die kontinuierliche Arbeitsweise.

Natürlich ist es auch möglich, die zugesetzte Hilfskomponente im wesentlichen vollständig vom 1,3-Cyclohexandion abzudestillieren, anstatt die Destillation bei dem obengenannten Restgehalt von 5 bis 50 Gew.-% zu beenden. In diesem Fall muß anschließend dem verbliebenen, mit Kristallen durchsetzten Konzentrat erneut eine — entweder die gleiche oder eine andere — der als Hilfskomponenten genannten Verbindungen oder Verbindungsgemische zugesetzt werden, damit sich das 1,3-Cyclohexandion möglichst vollständig kristallin abscheidet und ein filtrierbarer Kristallbrei entsteht.

Ein weiterer Gegenstand der vorliegenden Erfindung ist demgemäß ein Verfahren zur Isolierung von kristallinem 1,3-Cyclohexandion aus dem bei der katalytischen Gasphase-Cyclisierung von 5-Oxohexansäure gebildeten Rohprodukt, das dadurch gekennzeichnet ist, daß man dem Rohprodukt vor oder direkt nach der Kondensation 10 bis 100 Gew.-%, bezogen auf das Rohprodukt, einer Hilfskomponente zugibt, die aus mindestens einer Verbindung aus der von cyclischen Kohlenwasserstoffen, chlorierten aliphatischen Kohlenwasserstoffen und Monoethern gebildeten Gruppe besteht, dann die Mischung aus Rohprodukt und Hilfskomponente zumindest so lange unter normalem oder vermindertem Druck bei 0 bis 80° C destilliert, bis das Reaktionswasser entfernt ist, und dann das verbliebene Konzentrat mit einer solchen Menge einer Hilfskomponente der genannten Art mischt, daß deren Anteil an der Mischung 5 bis 50 Gew.-% beträgt und das danach ausgefallene kristalline 1,3-Cyclohexandion abfiltriert.

Dieses Verfahren wird im folgenden auch als »Zweistufen-Verfahren« bezeichnet, weil zweimal Hilfskomponente zugesetzt wird, im Gegensatz zu dem zuerst beschriebenen Verfahren, bei dem nur einmal Hilfskomponente zugesetzt wird und das daher im folgenden auch als »Einstufen-Verfahren« bezeichnet wird.

Vorzugsweise beträgt die vor oder direkt nach der Kondensation des Rohprodukts zugegebene Menge an Hilfskomponente beim Zweistufen-Verfahren 20 bis 80 Gew.-%, insbesondere 20 bis 60 Gew.-%, jeweils bezogen auf das Rohprodukt, wobei die optimale prozentuale Menge innerhalb dieser Grenzen sich wieder nach dem Wassergehalt des Rohprodukts und nach dem Wassergehalt des gebildeten Azeotrops richtet.

Bevorzugt sind wieder die bereits beim Einstufen-Verfahren als bevorzugt genannten Hilfskomponenten. Besonders bevorzugte Hilfskomponenten sind jetzt jedoch Toluol und Xylol.

Die Temperatur bei der anschließenden Destillation beträgt vorzugsweise 10 bis 40° C.

Dem hierbei entstandenen Konzentrat wird vorzugsweise so viel an Hilfskomponente zugesetzt, daß deren Anteil an der entstehenden Mischung 10 bis 30 Gew.-% beträgt.

Die Kondensation des gasförmigen Produktstroms wird vorteilhaft so durchgeführt wie beim Einstufen-Verfahren beschrieben.

Das Zweistufen-Verfahren hat den Vorteil, daß sich die für Wasserabtrennung und Abscheidung des Cyclohexandions optimalen Mengen an Hilfskomponente leichter bestimmen lassen als beim Einstufen-Verfahren.

Die beiden bisher beschriebenen Methoden gehen davon aus, daß der gasförmig aus dem Reaktor austretende Produktstrom in ein einheitliches Kondensat — entweder durch Kondensation in einem einzigen Gefäß oder durch Kondensation in mehreren hintereinandergeschalteten Gefäßen und anschließende Vereinigung der Kondensate — übergeführt wird, welches dann weiterverarbeitet wird. Man kann jedoch statt dessen den Produktstrom in bestimmter Weise in zwei Kondensate verschiedener Zusammensetzung überführen und diese voneinander getrennt weiterverarbeiten. Zu diesem Zweck kondensiert man das gasförmige Produkt in zwei oder mehr hintereinandergeschalteten Gefäßen, wobei die Temperaturen dieser Gefäße so eingestellt werden, daß das 1,3-Cyclohexandion hauptsächlich im ersten Gefäß, dagegen das Reaktionswasser im wesentlichen erst im zweiten Gefäß und ggf. den folgenden Gefäßen kondensiert. Dies wird erreicht, indem man die Temperatur des ersten Gefäßes auf 20 bis 80° C, vorzugsweise 20 bis 60° C, einstellt und die Temperatur des zweiten und ggf. der folgenden Gefäße auf —40 bis +60° C, vorzugsweise 0 bis 60° C. Falls man mehr als zwei Gefäße verwendet, wird im allgemeinen der Inhalt des zweiten Gefäßes mit dem Inhalt des oder der folgenden Gefäße vereinigt. Manchmal ist es jedoch vorteilhaft, den Inhalt des oder der auf das zweite folgenden Gefäße zu verwerfen oder doch zumindest den Inhalt der letzten in der Reihe der Gefäße, die hauptsächlich Wasser enthalten. Man erhält jedenfalls stets zwei Kondensate, ein erstes mit dem Inhalt des ersten Gefäßes und ein zweites Kondensat mit dem Inhalt des zweiten sowie ggf. des oder der folgenden Gefäße. Da das erste Kondensat im wesentlichen wasserfrei ist, entfällt die sonst notwendige Wasserentfernung; man setzt eine solche Menge einer Hilfskomponente der genannten Art zu, daß deren Anteil an der Mischung 5 bis 50 Gew.-% beträgt und filtriert das danach ausgefallene kristalline 1,3-Cyclohexandion ab. Das zweite Kondensat enthält den wesentlichen Teil des Reaktionswassers und wird durch ein- oder zweistufigen Zusatz einer Hilfskomponente und Wasserentfernung aufgearbeitet, wie zu Beginn geschildert, d. h., es wird genauso behandelt — inklusive der bevorzugten Maßnahmen — wie das obenerwähnte einheitliche Kondensat des gesamten Reaktionsproduktes.

Ein weiterer Gegenstand der vorliegenden Erfindung ist demgemäß ein Verfahren zur Isolierung von kristallinem 1,3-Cyclohexandion aus dem bei der katalytischen Gasphase-Cyclisierung von 5-Oxohexansäure gebildeten Rohprodukt, das dadurch gekennzeichnet ist, daß man aus dem gasförmigen Rohprodukt durch Abkühlung auf 20 bis 80° C ein erstes Kondensat und durch anschließende Abkühlung des noch verbliebenen Gases auf —40 bis +60° C ein zweites Kondensat gewinnt, dann das erste Kondensat mit einer solchen Menge einer Hilfskomponente mischt, daß deren Anteil an der Mischung 5 bis 50 Gew.-% beträgt, wobei die Hilfskomponente aus mindestens einer Verbindung aus der von cyclischen Kohlenwasserstoffen, chlorierten aliphatischen Kohlenwasserstoffen und Monoethern gebildeten Gruppe besteht, und das danach ausgefallene kristalline 1,3-Cyclohexandion abfiltriert, dann das zweite Kondensat mit 20 bis 200 Gew.-%, bezogen auf das Kondensat, einer Hilfskomponente der genannten Art mischt und dann die Mischung so lange unter normalem oder vermindertem Druck bei 0 bis 80°C destilliert, bis das Reaktionswasser entfernt ist und der Gehalt an Hilfskomponente im zurückbleibenden Konzentrat 5 bis 50 Gew.-% beträgt, und das danach ausgefallene kristalline 1,3-Cyclohexandion abfiltriert.

Ein weiterer Gegenstand der vorliegenden Erfindung ist schließlich ein Verfahren zur Isolierung von kristallinem 1,3-Cyclohexandion aus dem bei der katalytischen Gasphase-Cyclisierung von 5-Oxohexansäure gebildeten Rohprodukt, das dadurch gekennzeichnet ist, daß man aus dem gasförmigen Rohprodukt durch Abkühlung auf 20 bis 80°C ein erstes Kondensat und durch anschließende Abkühlung des noch verbliebenen Gases auf —40 bis +60°C ein zweites Kondensat gewinnt, dann das erste Kondensat mit einer solchen Menge einer Hilfskomponente mischt, daß deren Anteil an der Mischung 5 bis 50 Gew.-% beträgt, wobei die Hilfskomponente aus mindestens einer Verbindung aus der von cyclischen Kohlenwasserstoffen, chlorierten aliphatischen Kohlenwasserstoffen und Monoethern gebildeten Gruppe besteht und das danach ausgefallene kristalline 1,3-Cyclohexandion abfiltriert, dann das zweite Kondensat mit 10 bis 100 Gew.-%, bezogen auf das Kondensat, einer Hilfskomponente der genannten Art mischt und dann die Mischung zumindest so lange unter normalem oder vermindertem Druck bei 0 bis 80°C destilliert, bis das Reaktionswasser entfernt ist, und dann das verbliebene Konzentrat mit einer solchen Menge einer Hilfskomponente der genannten Art mischt, daß deren Anteil an der Mischung 5 bis 50 Gew.-% beträgt und das danach ausgefallene kristalline 1,3-Cyclohexandion abfiltriert.

Das nach einer der beschriebenen Methoden erhaltene kristalline 1,3-Cyclohexandion wird anschließend gewaschen, vorzugsweise mit einer der als Hilfskomponenten genannten Verbindungen oder Verbindungsgemischen. Besonders geeignet ist Toluol.

Bei den vier bisher erwähnten Arbeitsweisen wird das Rohprodukt der 5-Oxohexansäure-Cyclisierung vor oder direkt nach der Kondensation mit der Hilfskomponente vermischt.

Bei den vier folgenden Arbeitsweisen wird dagegen das Rohprodukt kondensiert und danach für eine gewisse Zeit erwärmt. Das dabei, gegebenenfalls nach Abkühlung, erhaltene sogenannte »Rohkristallisat« wird dann mit der Hilfskomponente vermischt und weiterverarbeitet.

Ein weiterer Gegenstand der vorliegenden Erfindung ist daher ein Verfahren zur Isolierung von kristallinem 1,3-Cyclohexandion aus dem bei der katalytischen Gasphase-Cyclisierung von 5-Oxohexansäure gebildeten Rohprodukt, das dadurch gekennzeichnet ist, daß man das Rohprodukt nach der Kondensation 0,5 bis 20 Stunden lang auf 20 bis 80°C erwärmt und das, ggf. nach Abkühlung, erhaltene Rohkristallisat anschließend mit 20 bis 150 Gew.-%, bezogen auf das Rohkristallisat, einer Hilfskomponente mischt, die aus mindestens einer Verbindung aus der von cyclischen Kohlenwasserstoffen, chlorierten aliphatischen Kohlenwasserstoffen und Monoethern gebildeten Gruppen besteht, dann die Mischung aus Rohkristallisat und Hilfskomponente so lange unter normalem oder vermindertem Druck bei 0 bis 80°C destilliert, bis das Reaktionswasser entfernt ist und der Gehalt an Hilfskomponente im zurückbleibenden Konzentrat 5 bis 50 Gew.-% beträgt, und das danach ausgefallene kristalline 1,3-Cyclohexandion abfiltriert. Vorzugsweise destilliert man bei 10 bis 40°C. Dabei sind die soeben und im folgenden angegebenen Destillationstemperaturen stets die im Sumpf gemessenen Temperaturen.

Das Rohprodukt wird auf 20 bis 80°C, vorzugsweise auf 20 bis 60°C, insbesondere auf 30 bis 50°C erwärmt. Die Dauer der Erwärmung beträgt 0,5 bis 20 Stunden, vorzugsweise 1 bis 12 Stunden, insbesondere 2 bis 10 Stunden.

Die als Hilfskomponente in Frage kommenden cyclischen Kohlenwasserstoffe können Aromaten oder Cycloaliphaten sowie chloriert oder unchloriert sein. Sie haben im allgemeinen bis zu 12 C-Atome. Bevorzugt sind Benzol, Toluol, Xylol, Mesitylen, Cumol, Ethylbenzol, Chlorbenzol, Dichlorbenzol, Tetralin, Dekalin, Cyclohexan und Methylcyclohexan. Besonders bevorzugt sind Xylol, Mesitylen, Cumol, Ethylbenzol, Chlorbenzol, Dichlorbenzol, Tetralin und Dekalin.

Die chlorierten aliphatischen Kohlenwasserstoffe haben im allgemeinen bis zu 6 C-Atome. Bevorzugt sind Methylenchlorid, Chloroform, Tetrachlorkohlenstoff, Dichlorethan, Tetrachlorethan und Butylchlorid. Besonders bevorzugt ist Tetrachlorethan.

Die Monoether können aliphatisch oder aromatisch sein. Sie haben im allgemeinen bis zu 12 C-Atome; bevorzugt sind Diisopropylether, Di-n-butylether und Di-n-hexylether, Anisol und Phenetol; besonders bevorzugt ist Di-n-butylether.

Die am besten geeigneten Hilfskomponenten sind Xylol und Di-n-butylether.

Die Hilfskomponente wird in Mengen von 20 bis 150 Gew.-%, vorzugsweise 20 bis 80 Gew.-%, insbesondere 20 bis 60 Gew.-%, bezogen auf das Rohkristallisat, zugesetzt, wobei die optimale prozentuale Menge sich innerhalb dieser Grenzen nach dem Wassergehalt des Rohkristallisats und nach dem Wassergehalt des Azeotrops aus Wasser und jeweiliger Hilfskomponente richtet. Zum Beispiel enthält ein Wasser-Xylol-Azeotrop unter sonst gleichen Bedingungen ca. 10mal so viel Wasser wie ein Wasser-Diisopropylether-Azeotrop.

Natürlich ist es auch möglich, die zugesetzte Hilfskomponente im wesentlichen vollständig vom 1,3-Cyclohexandion abzudestillieren, anstatt die Destillation bei dem obengenannten Restgehalt von 5 bis 50 Gew.-% zu beenden. In diesem Fall muß anschließend dem verbliebenen, mit Kristallen durchsetzten Konzentrat erneut eine — entweder die gleiche oder eine andere — der als Hilfskomponenten genannten Verbindungen oder Verbindungsgemische zugesetzt werden, damit sich das 1,3-Cyclohexandion möglichst vollständig kristallin abscheidet und ein filtrierbarer Kristallbrei entsteht.

Ein weiterer Gegenstand der vorliegenden Erfindung ist demgemäß ein Verfahren zur Isolierung von kristallinem 1,3-Cyclohexandion aus dem bei der katalytischen Gasphase-Cyclisierung von 5-Oxohexansäure gebildeten Rohprodukt, das dadurch gekennzeichnet ist, daß man das Rohprodukt nach der Kondensation 0,5 bis 20 Stunden lang auf 20 bis 80° C erwärmt und das, ggf. nach Abkühlung, erhaltene Rohkristallisat anschließend mit 10 bis 100 Gew.-%, bezogen auf das Rohkristallisat, einer Hilfskomponente mischt, die aus mindestens einer Verbindung aus der von cyclischen Kohlenwasserstoffen, chlorierten aliphatischen Kohlenwasserstoffen und Monoethern gebildeten Gruppe besteht, dann die Mischung aus Rohkristallisat und Hilfskomponente zumindest so lange unter normalen oder vermindertem Druck bei 0 bis 80° C destilliert, bis das Reaktionswasser entfernt ist, und dann das verbliebene Konzentrat mit einer solchen Menge einer Hilfskomponente der genannten Art mischt, daß deren Anteil an der Mischung 5 bis 50 Gew.-% beträgt und das danach ausgefallene kristalline 1,3-Cyclohexandion abfiltriert.

Dieses Verfahren wird im folgenden auch als »Rohkristallisat-Zweistufen-Verfahren« bezeichnet, weil zweimal Hilfskomponente zugesetzt wird, im Gegensatz zu dem davor beschriebenen Verfahren, bei dem nur einmal Hilfskomponente zugesetzt wird und das daher im folgenden auch als »Rohkristallisat-Einstufen-Verfahren« bezeichnet wird.

Vorzugsweise beträgt die nach der Kondensation des Rohprodukts zugegebene Menge an Hilfskomponente beim Rohkristallisat-Zweistufen-Verfahren 20 bis 70 Gew.-%, insbesondere 20 bis 50 Gew.-%, jeweils bezogen auf das Rohkristallisat, wobei die optimale prozentuale Menge innerhalb dieser Grenzen sich wieder nach dem Wassergehalt des Rohkristallisats und nach dem Wassergehalt des gebildeten Azeotrops richtet.

Bevorzugt sind wieder die bereits beim Rohkristallisat-Einstufen-Verfahren als bevorzugt genannten Hilfskomponenten. Besonders bevorzugte Hilfskomponenten sind jetzt jedoch Toluol und Xylol.

Die Temperatur bei der anschließenden Destillation beträgt vorzugsweise 10 bis 40° C.

Dem hierbei entstandenen Konzentrat wird vorzugsweise so viel an Hilfskomponente zugesetzt, daß deren Anteil an der entstehenden Mischung 10 bis 30 Gew.-% beträgt.

Das Rohkristallisat-Zweistufen-Verfahren hat den Vorteil, daß sich die für Wasserabtrennung und Abscheidung des Cyclohexandions optimalen Mengen an Hilfskomponente leichter bestimmen lassen als beim Rohkristallisat-Einstufen-Verfahren.

Die beiden soeben beschriebenen Methoden gehen davon aus, daß der gasförmig aus dem Reaktor austretende Produktstrom in ein einheitliches Kondensat — entweder durch Kondensation in einem einzigen Gefäß oder durch Kondensation in mehreren hintereinandergeschalteten Gefäßen und anschließende Vereinigung der Kondensate — übergeführt wird, welches dann weiterverarbeitet wird. Man kann jedoch statt dessen den Produktstrom in bestimmter Weise in zwei Kondensate verschiedener Zusammensetzung überführen und diese voneinander getrennt weiterverarbeiten. Zu diesem Zweck kondensiert man das gasförmige Produkt in zwei oder mehr hintereinandergeschalteten Gefäßen, wobei die Temperaturen dieser Gefäße so eingestellt werden, daß das 1,3-Cyclohexandion hauptsächlich im ersten Gefäß, dagegen das Reaktionswasser im wesentlichen erst im zweiten Gefäß und ggf. den folgenden Gefäßen kondensiert. Dies wird erreicht, indem man die Temperatur des ersten Gefäßes auf 20 bis 80° C, vorzugsweise 20 bis 60° C einstellt und die Temperatur des zweiten und ggf. der folgenden Gefäße auf —40 bis +60° C, vorzugsweise 0 bis 60° C. Falls man mehr als zwei Gefäße verwendet, wird im allgemeinen der Inhalt des zweiten Gefäßes mit dem Inhalt des oder der folgenden Gefäße vereinigt. Manchmal ist es jedoch vorteilhaft, den Inhalt des oder der auf das zweite folgenden Gefäße zu verwerfen oder doch zumindest den Inhalt der letzten in der Reihe der Gefäße, die hauptsächlich Wasser enthalten. Man erhält jedenfalls stets zwei Kondensate, ein erstes mit dem Inhalt des ersten Gefäßes und ein zweites Kondensat mit dem Inhalt des zweiten sowie ggf. des oder der folgenden Gefäße. Da das erste Kondensat im wesentlichen wasserfrei ist, entfällt die sonst notwendige Wasserentfernung; man setzt eine solche Menge einer Hilfskomponente der genannten Art zu, daß deren Anteil an der Mischung 5 bis 50 Gew.-% beträgt und filtriert das danach ausgefallene kristalline 1,3-Cyclohexandion ab. Das zweite Kondensat enthält den wesentlichen Teil des Reaktionswassers. Es wird, wie oben beschrieben, erwärmt und das erhaltene Rohkristallisat dann durch ein- oder zweistufigen Zusatz einer Hilfskomponente und Wasserentfernung aufgearbeitet, wie zu Beginn geschildert, d. h., es wird genauso behandelt — inklusive der bevorzugten Maßnahmen — wie das obenerwähnte einheitliche Kondensat des gesamten Reaktionsproduktes.

Ein weiterer Gegenstand der vorliegenden Erfindung ist demgemäß ein Verfahren zur Isolierung von kristallinem 1,3-Cyclohexandion aus dem bei der katalytischen Gasphase-Cyclisierung von 5-Oxohexansäure gebildeten Rohprodukt, das dadurch gekennzeichnet ist, daß man aus dem gasförmigen Rohprodukt durch Abkühlung auf 20 bis 80° C ein erstes Kondensat und durch anschließende Abkühlung des noch verbliebenen Gases auf −40 bis +60°C ein zweites Kondensat gewinnt, dann das erste Kondensat mit einer solchen Menge einer Hilfskomponente mischt, daß deren Anteil an der Mischung 5 bis 50 Gew.-% beträgt, wobei die Hilfskomponente aus mindestens einer Verbindung aus der von cyclischen Kohlenwasserstoffen, chlorierten aliphatischen Kohlenwasserstoffen und Monoethern gebildeten Gruppe besteht, und das danach ausgefallene kristalline 1,3-Cyclohexandion abfiltriert, dann das zweite Kondensat 0,5 bis 20 Stunden lang auf 20 bis 80°C erwärmt und das, ggf. nach Abkühlung, erhaltene Rohkristallisat anschließend mit 20 bis 200 Gew.-%, bezogen auf das Rohkristallisat, einer Hilfskomponente der genannten Art mischt und dann die Mischung so lange unter normalem oder vermindertem Druck bei 0 bis 80°C destilliert, bis das Reaktionswasser entfernt ist und der Gehalt an Hilfskomponente im zurückbleibenden Konzentrat 5 bis 50 Gew.-% beträgt, und das danach ausgefallene kristalline 1,3-Cyclohexandion abfiltriert.

Ein weiterer Gegenstand der vorliegenden Erfindung ist 1,3-Cyclohexandion aus dem bei der katalytischen Gasphase-Cyclisierung von 5-Oxohexansäure gebildeten Rohprodukt, das dadurch gekennzeichnet ist, daß man aus dem gasförmigen Rohprodukt durch Abkühlung auf 20 bis 80°C ein erstes Kondensat und durch anschließende Abkühlung des noch verbliebenen Gases auf −40 bis +60°C ein zweites Kondensat gewinnt, dann das erste Kondensat mit einer solchen Menge einer Hilfskomponente mischt, daß deren Anteil an der Mischung 5 bis 50 Gew.-% beträgt, wobei die Hilfskomponente aus mindestens einer Verbindung aus der von cyclischen Kohlenwasserstoffen, chlorierten aliphatischen Kohlenwasserstoffen und Monoethern gebildeten Gruppe besteht und das danach ausgefallene kristalline 1,3-Cyclohexandion abfiltriert, dann das zweite Kondensat 0,5 bis 20 Stunden lang auf 20 bis 80°C erwärmt und das, ggf. nach Abkühlung, erhaltene Rohkristallisat anschließend mit 10 bis 100 Gew.-%, bezogen auf das Rohkristallisat, einer Hilfskomponente der genannten Art mischt und dann die Mischung zumindest so lange unter normalem oder vermindertem Druck bei 0 bis 80°C destilliert, bis das Reaktionswasser entfernt ist, und dann das verbliebene Konzentrat mit einer solchen Menge einer Hilfskomponente der genannten Art mischt, daß deren Anteil an der Mischung 5 bis 50 Gew.-% beträgt, und das danach ausgefallene kristalline 1,3-Cyclohexandion abfiltriert.

Das nach einer der beschriebenen Methoden erhaltene kristalline 1,3-Cyclohexandion wird anschließend gewaschen, vorzugsweise mit einer der als Hilfskomponenten genannten Verbindungen oder Verbindungsgemischen. Besonders geeignet ist Toluol.

Die nachfolgenden Beispiele sollen die Erfindung näher erläutern. Die angegebenen Prozentzahlen sind stets Gewichtsprozente.

### Beispiel 1

5-Oxohexansäure wird im Stickstoffstrom bei 380°C über ein kristallines Alumosilikat mit einer LHSV (h$^{-1}$) von 2,0 geleitet. Das Molverhältnis 5-Oxohexansäure zu N$_2$ ist dabei 1 : 4. Das Rohprodukt (1,3 kg, Cyclohexandionanteil 40%) wird in 500 ml (das sind 33,5%, bezogen auf das Rohprodukt) Toluol aufgenommen und diese Mischung in einen 4-l-Vierhalskolben gebracht, der mit Rührer und absteigendem Kühler versehen ist. Diese Mischung hat folgende Zusammensetzung: 25% Toluol, 30% 1,3-Cyclohexandion, 14% 6-Methyl-3,4-dihydro-2-pyranon, 8% Wasser, 18% 5-Oxohexansäure und ca. 5% Nebenprodukte. Bei ca. 3 mbar und 40°C Badtemperatur wird zunächst Wasser mit Toluol unter Rühren so lange abdestilliert, bis die Sumpftemperatur gegen Ende der Destillation auf 35°C ansteigt.

Anschließend werden in den hellgelben, teilweise kristallinen Rückstand (990 g) bei Raumtemperatur 285 ml Toluol eingerührt. Die erhaltene Suspension läßt man bei 0°C 4 bis 5 Stunden stehen und saugt dann den Kristallbrei über eine Glasfilternutsche scharf ab (ca. 20 Minuten lang).

Der Filterkuchen wird zunächst auf der Nutsche mit 250 ml Toluol überschichtet und nochmals scharf abgesaugt. Dann wird er mit 600 ml Toluol intensiv aufgerührt, die Waschflüssigkeit wiederum abgesaugt und anschließend dieser Vorgang mit der gleichen Toluolmenge wiederholt.

Das nahezu farblose Produkt wiegt nach Trocknung 444 g, der Cyclohexandiongehalt beträgt 98,8%, der Oxohexansäuregehalt 0,6%. Damit werden 84,4% des im Rohprodukt vorhandenen 1,3-Cyclohexandions in kristalliner Form isoliert.

### Beispiel 2

5-Oxohexansäure wird bei 360°C, aber ansonsten wie in Beispiel 1, umgesetzt. Das Rohprodukt (1,3 kg, Cyclohexandionanteil 34%) wird in 1000 ml (das sind 59,2%, bezogen auf das Rohprodukt) Dibutylether aufgenommen und die Mischung in einen 4-l-Vierhalskolben gebracht, der mit Rührer und absteigendem Kühler ausgerüstet ist. Diese Mischung hat folgende Zusammensetzung: 37,5% Dibutylether, 21% 1,3-Cyclohexandion, 10% 6-Methyl-3,4-dihydro-2-pyranon, 5% Wasser, 22% 5-Oxohexansäure und ca. 4% Nebenprodukte. Aus dieser Mischung werden 800 ml Dibutylether zusammen mit dem Reaktionswasser bei ca. 10 mbar, 50°C Badtemperatur und maximal 40°C Sumpftemperatur abdestilliert.

Den hellgelben, teilweise kristallinen Rückstand (1190 g) läßt man bei 0°C 4 bis 5 Stunden stehen und saugt dann den Kristallbrei über eine Glasfilternutsche scharf ab (ca. 20 Min. lang).

Der Filterkuchen wird zunächst auf der Nutsche mit 200 ml Toluol überschichtet und nochmals scharf abgesaugt. Dann wird er mit 500 ml Toluol intensiv aufgerührt, die Waschflüssigkeit wiederum abgesaugt und anschließend dieser Vorgang mit der gleichen Toluolmenge wiederholt.

Das nahezu farblose Produkt wiegt nach Trocknung 336 g, der Cyclohexandiongehalt beträgt 99,1%, der Oxohexansäuregehalt 0,4%. Damit werden 75,3% des im Rohprodukt vorhandenen 1,3-Cyclohexandions in kristalliner Form isoliert.

Vergleichsbeispiel 1

250 g Rohprodukt (Cyclohexandionanteil 40%), die nach sechsstündigem Stehen durchkristallisiert sind, werden durch Umrühren zu einem Kristallbrei homogenisiert, der anschließend über eine Glasfilternutsche scharf abgesaugt wird. Infolge der hohen Viskosität der Mutterlauge erfordert dies einen Zeitaufwand von 3—4 Stunden. Der kristalline Filterkuchen wird mit 70 ml Toluol aufgerührt, nochmals abgesaugt und die Wäsche mit der gleichen Toluolmenge wiederholt.

Das gelblich gefärbte kristalline Produkt wiegt nach Trocknung 45 g, der Cyclohexandiongehalt beträgt 94%. Damit können nur 42,3% des im Rohprodukt vorhandenen 1,3-Cyclohexandions in dieser zudem verunreinigten Form isoliert werden; das Produkt enthält noch 5% 5-Oxohexansäure.

Vergleichsbeispiel 2

Man verfährt wie in Vergleichsbeispiel 1, nur daß der kristalline Filterkuchen zweimal mit 70 ml Tetrachlorkohlenstoff statt Toluol gewaschen wird.

Das gelblich gefärbte kristalline Produkt wiegt nach Trocknung 59 g, der Cyclohexandiongehalt beträgt 82%. Damit können nur 48,3% des im Rohprodukt vorhandenen 1,3-Cyclohexandions in dieser verunreinigten Form isoliert werden; das Produkt enthält noch 16% 5-Oxohexansäure.

Vergleichsbeispiel 3

Man verfährt wie in Beispiel 1, jedoch wird das in Toluol aufgenommene Rohprodukt nicht der Entwässerung unterworfen, sondern bei Raumtemperatur aufbewahrt. Nach mehrtägigem Stehen ist noch keine Abscheidung von Kristallen erfolgt.

Beispiel 3

5-Oxohexansäure wird im Stickstoffstrom bei 380°C über ein kristallines Alumosilikat mit einer LHSV (h$^{-1}$) von 2,0 geleitet. Das Molverhältnis 5-Oxohexansäure : N$_2$ ist dabei 1 : 4. Das Rohprodukt (1,3 kg, Cyclohexandionanteil 40%) wird unter Rühren 4 Stunden bei 40°C gehalten und anschließend mit 375 ml Toluol vermischt (das sind 25%, bezogen auf das Rohprodukt). Diese Mischung hat folgende Zusammensetzung: 20% Toluol, 32% 1,3-Cyclohexandion, 0,5% 6-Methyl-3,4-dihydro-2-pyranon, 5% Wasser, 38% 5-Oxohexansäure und ca. 4% Nebenprodukte. Bei ca. 3 mbar und 45°C Badtemperatur wird zunächst Wasser mit Toluol unter Rühren so lange abdestilliert, bis die Sumpftemperatur gegen Ende der Destillation auf 38°C ansteigt.

Anschließend werden in den hellgelben, teilweise kristallinen Rückstand (1085 g) bei Raumtemperatur 325 ml Toluol eingerührt. Die erhaltene Suspension läßt man bei 0°C 4 bis 5 Stunden stehen und saugt dann den Kristallbrei über eine Glasfilternutsche scharf ab (ca. 40 Min. lang).

Der Filterkuchen wird zunächst auf der Nutsche mit 250 ml Toluol überschichtet und nochmals scharf abgesaugt. Dann wird er mit 500 ml Toluol intensiv aufgerührt, die Waschflüssigkeit wiederum abgesaugt und anschließend dieser Vorgang mit der gleichen Toluolmenge wiederholt.

Das nahezu farblose Produkt wiegt nach Trocknung 377 g, der Cyclohexandiongehalt beträgt 98,2%, der Oxohexansäuregehalt 1,4%. Damit werden 71,2% des im Rohprodukt vorhandenen 1,3-Cyclohexandions in kristalliner Form isoliert.

Beispiel 4

5-Oxohexansäure wird wie in Beispiel 3 umgesetzt. Das Rohprodukt (1,3 kg, Cyclohexandionanteil 40%) wird unter Rühren 4 Stunden bei 40°C gehalten und anschließend mit 850 ml Xylol (das sind 57,6%, bezogen auf das Rohprodukt) vermischt. Diese Mischung hat folgende Zusammensetzung: 36,5% Xylol, 25,4% 1,3-Cyclohexandion, 0,6% 6-Methyl-3,4-dihydro-2-pyranon, 4% Wasser, 29% 5-Oxohexansäure und ca. 4% Nebenprodukte.

Aus dieser Mischung werden 600 ml Xylol zusammen mit dem Reaktionswasser bei ca. 10 mbar, 45°C Badtemperatur und max. 40°C Sumpftemperatur abdestilliert.

Den hellgelben, teilweise kristallinen Rückstand (1215 g) läßt man bei 0°C 4 bis 5 Stunden stehen und saugt dann den Kristallbrei über eine Glasfilternutsche scharf ab (ca. 45 Min. lang).

Der Filterkuchen wird zunächst auf der Nutsche mit 200 ml Toluol überschichtet und nochmals scharf abgesaugt. Dann wird er mit 550 ml Toluol intensiv aufgerührt, die Waschflüssigkeit wiederum abgesaugt und anschließend dieser Vorgang mit der gleichen Toluolmenge wiederholt.

Das nahezu farblose Produkt wiegt nach Trocknung 382 g, der Cyclohexandiongehalt beträgt 98,3%, der Oxohexansäuregehalt 1,2%. Damit werden 72,2% des im Rohprodukt vorhandenen 1,3-Cyclohexandions in kristalliner Form isoliert.

## Vergleichsbeispiel 4

250 g Rohprodukt (Cyclohexandionanteil 40%), die nach sechsstündigem Stehen durchkristallisiert sind, werden durch Umrühren zu einem Kristallbrei homogenisiert, der anschließend über eine Glasfilternutsche scharf abgesaugt wird. Infolge der hohen Viskosität der Mutterlauge erfordert dies einen Zeitaufwand von 3—4 Stunden. Der kristalline Filterkuchen wird mit 70 ml Toluol aufgerührt, nochmals abgesaugt und die Wäsche mit der gleichen Toluolmenge wiederholt.

Das gelblich gefärbte kristalline Produkt wiegt nach Trocknung 45 g, der Cyclohexandiongehalt beträgt 94%. Damit können nur 42,3% des im Rohprodukt vorhandenen 1,3-Cyclohexandions in dieser zudem verunreinigten Form isoliert werden; das Produkt enthält noch 5% 5-Oxohexansäure.

## Vergleichsbeispiel 5

Man verfährt wie in Vergleichsbeispiel 4, nur daß der kristalline Filterkuchen zweimal mit 70 ml Tetrachlorkohlenstoff statt Toluol gewaschen wird.

Das gelblich gefärbte kristalline Produkt wiegt nach Trocknung 59 g, der Cyclohexandiongehalt beträgt 82%. Damit können nur 48,3% des im Rohprodukt vorhandenen 1,3-Cyclohexandions in dieser verunreinigten Form isoliert werden; das Produkt enthält noch 16% 5-Oxohexansäure.

**Patentansprüche**

1. Verfahren zur Isolierung von kristallinem 1,3-Cyclohexandion aus dem bei der katalytischen Gasphase-Cyclisierung von 5-Oxohexansäure gebildeten Rohprodukt, dadurch gekennzeichnet, daß man dem Rohprodukt vor oder direkt nach der Kondensation 20 bis 200 Gew.-%, bezogen auf das Rohprodukt, einer Hilfskomponente zugibt, die aus mindestens einer Verbindung aus der von cyclischen Kohlenwasserstoffen, chlorierten aliphatischen Kohlenwasserstoffen und Monoethern gebildeten Gruppe besteht, dann die Mischung aus Rohprodukt und Hilfskomponente so lange unter normalem oder vermindertem Druck bei 0 bis 80° C destilliert, bis das Reaktionswasser entfernt ist und der Gehalt an Hilfskomponente im zurückbleibenden Konzentrat 5 bis 50 Gew.-% beträgt, und das danach ausgefallene kristalline 1,3-Cyclohexandion abfiltriert.

2. Verfahren zur Isolierung von kristallinem 1,3-Cyclohexandion aus dem bei der katalytischen Gasphase-Cyclisierung von 5-Oxohexansäure gebildeten Rohprodukt, dadurch gekennzeichnet, daß man dem Rohprodukt vor oder direkt nach der Kondensation 10 bis 100 Gew.-%, bezogen auf das Rohprodukt, einer Hilfskomponente zugibt, die aus mindestens einer Verbindung aus der von cyclischen Kohlenwasserstoffen, chlorierten aliphatischen Kohlenwasserstoffen und Monoethern gebildeten Gruppe besteht, dann die Mischung aus Rohprodukt und Hilfskomponente zumindest so lange unter normalem oder vermindertem Druck bei 0 bis 60° C destilliert, bis das Reaktionswasser entfernt ist, und dann das verbliebene Konzentrat mit einer solchen Menge einer Hilfskomponente der genannten Art mischt, daß deren Anteil an der Mischung 5 bis 50 Gew.-% beträgt, und das danach ausgefallene kristalline 1,3-Cyclohexandion abfiltriert.

3. Verfahren zur Isolierung von kristallinem 1,3-Cyclohexandion aus dem bei der katalytischen Gasphase-Cyclisierung von 5-Oxohexansäure gebildeten Rohprodukt, dadurch gekennzeichnet, daß man aus dem gasförmigen Rohprodukt durch Abkühlung auf 20 bis 80° C ein erstes Kondensat und durch anschließende Abkühlung des noch verbliebenen Gases auf —40 bis +60° C ein zweites Kondensat gewinnt, dann das erste Kondensat mit einer solchen Menge einer Hilfskomponente mischt, daß deren Anteil an der Mischung 5—50 Gew.-% beträgt, wobei die Hilfskomponente aus mindestens einer Verbindung aus der von cyclischen Kohlenwasserstoffen, chlorierten aliphatischen Kohlenwasserstoffen und Monoethern gebildeten Gruppe besteht, und das danach ausgefallene kristalline 1,3-Cyclohexandion abfiltriert, dann das zweite Kondensat mit 20 bis 200 Gew.-%, bezogen auf das Kondensat, einer Hilfskomponente der genannten Art mischt und dann die Mischung so lange unter normalem oder vermindertem Druck bei 0 bis 80° C destilliert, bis das Reaktionswasser entfernt ist und der Gehalt an Hilfskomponente im zurückbleibenden Konzentrat 5 bis 50 Gew.-% beträgt, und das danach ausgefallene kristalline 1,3-Cyclohexandion abfiltriert.

4. Verfahren zur Isolierung von kristallinem 1,3-Cyclohexandion aus dem bei der katalytischen Gasphase-Cyclisierung von 5-Oxohexansäure gebildeten Rohprodukt, dadurch gekennzeichnet, daß man aus dem gasförmigen Rohprodukt durch Abkühlung auf 20 bis 80° C ein erstes Kondensat und durch anschließende Abkühlung des noch verbliebenen Gases auf —40 bis +60° C ein zweites Kondensat gewinnt, dann das erste Kondensat mit eine solchen Menge einer Hilfskomponente mischt, daß deren Anteil an der Mischung 5—50 Gew.-% beträgt, wobei die Hilfskomponente aus mindestens einer Verbindung aus der von cyclischen Kohlenwasserstoffen, chlorierten aliphatischen Kohlenwasserstoffen und Monoethern gebildeten Gruppe besteht, und das danach ausgefallene kristalline 1,3-Cyclohexandion abfiltriert, dann das zweite Kondensat mit 10 bis 100 Gew.-%, bezogen auf das Kondensat, einer Hilfskomponente der genannten Art mischt und dann die Mischung zumindest so lange unter normalem oder vermindertem Druck bei 0 bis 80° C destilliert, bis das Reaktionswasser entfernt ist, und dann das verbliebene Konzentrat mit einer solchen Menge einer Hilfskomponente der genannten Art mischt, daß deren Anteil an der Mischung 5 bis 50 Gew.-% beträgt, und das danach ausgefallene kristalline 1,3-Cyclohexandion abfiltriert.

5. Verfahren nach Anspruch 1 oder 3, dadurch gekennzeichnet, daß man Xylol oder Di-n-butylether als Hilfskomponente verwendet.

6. Verfahren nach Anspruch 2 oder 4, dadurch gekennzeichnet, daß man Xylol oder Toluol als Hilfskomponente verwendet.

7. Verfahren nach Anspruch 1 oder 2, dadurch gekennzeichnet, daß man das gasförmige Rohprodukt unmittelbar nach dessen Kondensation mit der Hilfskomponente vermischt oder in der vorgelegten Hilfskomponente kondensiert.

8. Verfahren nach Anspruch 1 oder 2, dadurch gekennzeichnet, daß man in kontinuierlicher Fahrweise das gasförmige Rohprodukt und die flüssige Hilfskomponente am Reaktorausgang zusammenführt und die Mischung anschließend vollständig kondensiert.

9. Verfahren zur Isolierung von kristallinem 1,3-Cyclohexandion aus dem bei der katalytischen Gasphase-Cyclisierung von 5-Oxohexansäure gebildeten Rohprodukt, dadurch gekennzeichnet, daß man das Rohprodukt nach der Kondensation 0,5 bis 20 Stunden lang auf 20 bis 80° C erwärmt und das, ggf. nach Abkühlung, erhaltene Rohkristallisat anschließend mit 20 bis 150 Gew.-%, bezogen auf das Rohkristallisat, einer Hilfskomponente mischt, die aus mindestens einer Verbindung aus der von cyclischen Kohlenwasserstoffen, chlorierten aliphatischen Kohlenwasserstoffen und Monoethern gebildeten Gruppe besteht, dann die Mischung aus Rohkristallisat und Hilfskomponente so lange unter normalem oder vermindertem Druck bei 0 bis 80° C destilliert, bis das Reaktionswasser entfernt ist und der Gehalt an Hilfskomponente im zurückbleibenden Konzentrat 5 bis 50 Gew.-% beträgt, und das danach ausgefallene kristalline 1,3-Cyclohexandion abfiltriert.

10. Verfahren zur Isolierung von kristallinem 1,3-Cyclohexandion aus dem bei der katalytischen Gasphase-Cyclisierung von 5-Oxohexansäure gebildeten Rohprodukt, dadurch gekennzeichnet, daß man das Rohprodukt nach der Kondensation 0,5 bis 20 Stunden lang auf 20 bis 80° C erwärmt und das, ggf. nach Abkühlung, erhaltene Rohkristallisat anschließend mit 10 bis 100 Gew.-%, bezogen auf das Rohkristallisat, einer Hilfskomponente mischt, die aus mindestens einer Verbindung aus der von cyclischen Kohlenwasserstoffen, chlorierten aliphatischen Kohlenwasserstoffen und Monoethern gebildeten Gruppe besteht, dann die Mischung aus Rohkristallisat und Hilfskomponente zumindest so lange unter normalem oder vermindertem Druck bei 0 bis 80° C destilliert, bis das Reaktionswasser entfernt ist, und dann das verbliebene Konzentrat mit einer solchen Menge einer Hilfskomponente der genannten Art mischt, daß deren Anteil an der Mischung 5 bis 50 Gew.-% beträgt, und das danach ausgefallene kristalline 1,3-Cyclohexandion abfiltriert.

11. Verfahren zur Isolierung von kristallinem 1,3-Cyclohexandion aus dem bei der katalytischen Gasphase-Cyclisierung von 5-Oxohexansäure gebildeten Rohprodukt, dadurch gekennzeichnet, daß man aus dem gasförmigen Rohprodukt durch Abkühlung auf 20 bis 80° C ein erstes Kondensat und durch anschließende Abkühlung des noch verbliebenen Gases auf —40 bis +60° C ein zweites Kondensat gewinnt, dann das erste Kondensat mit einer solchen Menge einer Hilfskomponente mischt, daß deren Anteil an der Mischung 5 bis 50 Gew.-% beträgt, wobei die Hilfskomponente aus mindestens einer Verbindung aus der von cyclischen Kohlenwasserstoffen, chlorierten aliphatischen Kohlenwasserstoffen und Monoethern gebildeten Gruppe besteht, und das danach ausgefallene kristalline 1,3-Cyclohexandion abfiltriert, dann das zweite Kondensat 0,5 bis 20 Stunden lang auf 20 bis 80° C erwärmt und das, ggf. nach Abkühlung, erhaltene Rohkristallisat anschließend mit 20 bis 150 Gew.-%, bezogen auf das Rohkristallisat, einer Hilfskomponente der genannten Art mischt und dann die Mischung so lange unter normalem oder vermindertem Druck bei 0 bis 80° C destilliert, bis das Reaktionswasser entfernt ist und der Gehalt an Hilfskomponente im zurückbleibenden Konzentrat 5 bis 50 Gew.-% beträgt, und das danach ausgefallene kristalline 1,3-Cyclohexandion abfiltriert.

12. Verfahren zur Isolierung von kristallinem 1,3-Cyclohexandion aus dem bei der katalytischen Gasphase-Cyclisierung von 5-Oxohexansäure gebildeten Rohprodukt, dadurch gekennzeichnet, daß man aus dem gasförmigen Rohprodukt durch Abkühlung auf 20 bis 80° C ein erstes Kondensat und durch anschließende Abkühlung des noch verbliebenen Gases auf —40 bis +60° C ein zweites Kondensat gewinnt, dann das erste Kondensat mit einer solchen Menge einer Hilfskomponente mischt, daß deren Anteil an der Mischung 5 bis 50 Gew.-% beträgt, wobei die Hilfskomponente aus mindestens einer Verbindung aus der von cyclischen Kohlenwasserstoffen, chlorierten aliphatischen Kohlenwasserstoffen und Monoethern gebildeten Gruppe besteht, und das danach ausgefallene kristalline 1,3-Cyclohexandion abfiltriert, dann das zweite Kondensat 0,5 bis 20 Stunden lang auf 20 bis 80° C erwärmt und das, ggf. nach Abkühlung, erhaltene Rohkristallisat anschließend mit 10 bis 100 Gew.-%, bezogen auf das Rohkristallisat, einer Hilfskomponente der genannten Art mischt und dann die Mischung zumindest so lange unter normalem oder vermindertem Druck bei 0 bis 80° C destilliert, bis das Reaktionswasser entfernt ist, und dann das verbliebene Konzentrat mit einer solchen Menge einer Hilfskomponente der genannten Art mischt, daß deren Anteil an der Mischung 5 bis 50 Gew.-% beträgt, und das danach ausgefallene kristalline 1,3-Cyclohexandion abfiltriert.

13. Verfahren nach Anspruch 9 oder 11, dadurch gekennzeichnet, daß man Xylol oder Di-n-butylether als Hilfskomponente verwendet.

14. Verfahren nach Anspruch 10 oder 12, dadurch gekennzeichnet, daß man Xylol oder Toluol als Hilfskomponente verwendet.

## Claims

1. A process for the isolation of crystalline 1,3-cyclohexanedione from the crude product formed in the catalytic gaseous phase cyclization of 5-oxohexanoic acid, characterized by adding to the crude product before or directly after condensation from to 20 to 200 weight-%, relative to the crude product, of an auxiliary component consisting of at least one compound selected from the group consisting of cyclic hydrocarbons, chlorinated aliphatic hydrocarbons and monoethers; distilling then the mixture of crude product and auxiliary component at 0 to 80°C under normal or reduced pressure until the water of reaction is removed and the content of auxiliary component in the remaining concentrate is from 5 to 50 weight-%; and filtering off the precipitated crystalline 1,3-cyclohexanedione.

2. A process for the isolation of crystalline 1,3-cyclohexanedione from the crude product formed in the catalytic gaseous phase cyclization of 5-oxohexanoic acid, characterized by adding to the crude product before or directly after condensation from 10 to 100 weight-%, relative to the crude product, of an auxiliary component consisting of a least one compound selected from the group consisting of cyclic hydrocarbons, chlorinated aliphatic hydrocarbons and monoethers; distilling then the mixture of crude product and auxiliary component at 0 to 80°C under normal or reduced pressure at least until the water of reaction is removed, then mixing the remaining concentrate with such an amount of an auxiliary component of the cited kind that the proportion of the latter in the mixture is from 5 to 50 weight-%, and filtering off the precipitated crystalline 1,3-cyclohexanedione.

3. A process for the isolation of crystalline 1,3-cyclohexanedione from the crude product formed in the gaseous phase cyclization of 5-oxohexanoic acid, characterized by obtaining a first condensate from the gaseous crude product by cooling to 20 to 80°C and a second condensate by subsequent cooling of the remaining gas to −40 to +60°C, mixing then the first condensate with such an amount of an auxiliary component that the proportion of the latter in the mixture is from 5 to 50 weight-%; the auxiliary component consisting of at least one compound selected from the group consisting of cyclic hydrocarbons, chlorinated aliphatic hydrocarbons and monoethers; and filtering off the precipitated crystalline 1,3-cyclohexanedione, subsequently mixing the second condensate with 20 to 200 weight-%, relative to the condensate, of an auxiliary component of the above kind, and distilling the mixture at 0 to 80°C under normal or reduced pressure until the water of reaction is removed and the content of auxiliary component in the remaining concentrate is from 5 to 50 weight-%,

and filtering off the precipitated crystalline 1,3-cyclohexanedione.

4. A process for the isolation of crystalline 1,3-cyclohexanedione from the crude product formed in the catalytic gaseous phase cyclization of 5-oxohexanoic acid, characterized by obtaining a first condensate from the gaseous crude product by cooling to 20 to 80°C and a second condensate by subsequent cooling of the remaining gas to −40 to +60°C, mixing then the first condensate with such an amount of an auxiliary component that the proportion of the latter in the mixture is from 5 to 50 weight-%; the auxiliary component consisting of at least one compound selected from the group consisting of cyclic hydrocarbons, chlorinated aliphatic hydrocarbons and monoethers; and filtering off the precipitated crystalline 1,3-cyclohexanedione, subsequently mixing the second condensate with 10 to 100 weight-%, relative to the condensate, of an auxiliary component of the above kind, and distilling the mixture at 0 to 80°C under normal or reduced pressure at least until the water of reaction is removed, mixing then the remaining concentrate with such an amount of an auxiliary component of the cited kind that the proportion of the latter in the mixture is from 5 to 50 weight-%, and filtering off the precipitated crystalline 1,3-cyclohexanedione.

5. The process as claimed in claim 1 or 3, characterized by using xylene or di-n-butyl ether as auxiliary component.

6. The process as claimed in claim 2 or 4, characterized by using xylene or toluene as auxiliary component.

7. The process as claimed in claim 1 or 2, characterized by mixing the gaseous crude product directly after condensation with the auxiliary component, or condensing it in the auxiliary component.

8. The process as claimed in claim 1 or 2, characterized by uniting the gaseous crude product and the liquid component continuously at the reactor outlet, and subsequently condensing the mixture completely.

9. A process for the isolation of crystalline 1,3-cyclohexanedione from the crude product formed in the catalytic gaseous phase cyclization of 5-oxohexanoic acid, characterized by heating the crude product after the condensation for 0.5 to 20 hours at 20 to 80°C, mixing subsequently the crude crystallized product so obtained, optionally after cooling, with 20 to 150 weight-%, relative to the crude crystallized product, of an auxiliary component consisting of at least one compound selected from the group consisting of cyclic hydrocarbons, chlorinated aliphatic hydrocarbons and monoethers; distilling then the mixture of crude crystalline product and auxiliary component at 0 to 80°C under normal or reduced pressure until the water of reaction is removed and the content of auxiliary component in the remaining concentrate is from 5 to 50 weight-%; and filtering off the precipitated crystalline 1,3-cyclohexanedione.

10. A process for the isolation of crystalline 1,3-cyclohexanedione from the crude product formed in the catalytic gaseous phase cyclization of 5-oxohexanoic acid, characterized by heating the crude product after the condensation for 0.5 to 20 hours at 20 to 80°C, mixing subsequently the crude crystallized product so obtained, optionally after cooling, with 10 to 100 weight-%, relative to the crude crystallized product, of an auxiliary component consisting of at least one compound selected from the group consisting of cyclic hydrocarbons, chlorinated aliphatic hydrocarbons and monoethers; distilling then the mixture of crude crystallized product and auxiliary component at 0 to 80°C under normal or reduced pressure at least until the water of reaction is removed, then mixing the remaining concentrate with such an amount of an auxiliary component of the cited kind that the proportion of the latter in the mixture is from 5 to 50 weight-%, and filtering off the precipitated crystalline 1,3-cyclohexanedione.

11. A process for the isolation of crystalline 1,3-cyclohexanedione from the crude product formed in the gaseous phase cyclization of 5-oxohexanoic acid, characterized by obtaining a first condensate from the gaseous crude product by cooling to 20 to 80°C and a second condensate by subsequent cooling of the remaining gas to —40 to +60°C, mixing then the first condensate with such an amount of an auxiliary component that the proportion of the latter in mixture is from 5 to 50 weight-%; the auxiliary component consisting of at least one compound selected from the group consisting of cyclic hydrocarbons, chlorinated aliphatic hydrocarbons and monoethers; and filtering off the precipitated crystalline 1,3-cyclohexanedione, subsequently heating the second condensate for 0.5 to 20 hours to 20 to 80°C, mixing the crude crystallized product, optionally after cooling, with 20 to 150 weight-%, relative to the crude crystallized product, of an auxiliary component of the above kind, and distilling the mixture at 0 to 80°C under normal or reduced pressure until the water of reaction is removed and the content of auxiliary component in the remaining concentrate is from 5 to 50 weight-%, and filtering off the precipitated crystalline 1,3-cyclohexanedione.

12. A process for the isolation of crystalline 1,3-cyclohexanedione from the crude product formed in the catalytic gaseous phase cyclization of 5-oxohexanoic acid, characterized by obtaining a first condensate from the gaseous crude product by cooling to 20 to 80°C and a second condensate by subsequent cooling of the remaining gas to —40 to +60°C, mixing then the first condensate with such an amount of an auxiliary component that the proportion of the latter in the mixture is from 5 to 50 weight-%; the auxiliary component consisting of at least one compound selected from the group consisting of cyclic hydrocarbons, chlorinated aliphatic hydrocarbons and monoethers; and filtering off the precipitated crystalline 1,3-cyclohexanedione, subsequently heating the second condensate for 0.5 to 20 hours to 20 to 80°C, mixing the crude crystallized product, optionally after cooling, with 10 to 100 weight-%, relative to the crude crystallized product, of an auxiliary component of the above kind, and distilling the mixture at 0 to 80°C under normal or reduced pressure at least until the water of reaction is removed, mixing then the remaining concentrate with such an amount of an auxiliary component of the cited kind that the proportion of the latter in the mixture is from 5 to 50 weight-%, and filtering off the precipitated crystalline 1,3-cyclohexanedione.

13. The process as claimed in claim 9 or 11, using xylene or di-n-butyl ether as auxiliary component.

14. The process as claimed in claim 10 or 12, using xylene or toluene as auxiliary component.


**Revendications**

1. Un procédé pour isoler la 1,3-cyclohexanedione cristallisée du produit brut formé par cyclisation catalytique en phase gazeuse du 5-oxohexanoïque, procédé caractérisé en ce que l'on ajoute au produit brut, avant ou aussitôt après la condensation, 20 à 200% de son poids d'une composante auxiliaire comprenant un ou plusieurs composés choisis parmi des hydrocarbures cycliques, des hydrocarbures aliphatiques chlorés et des monoéthers, puis on distille le mélange ainsi obtenu à 0 à 80°C, à la pression normale ou sous pression réduite, jusqu'à ce que l'eau de réaction soit éliminée et que la teneur du concentré restant en composante auxiliaire soit de 5 à 50% en poids, et on sépare par filtration la 1,3-cyclohexane-dione ayant cristallisé.

2. Procédé pour isoler la 1,3-cyclohexanedione cristallisée du produit brut formé par cyclisation catalytique en phase gazeuse du 5-oxohexanoïque, procédé caractérisé en ce que l'on ajoute au produit brut, avant ou aussitôt après la condensation, 10 à 100% de son poids d'une composante auxiliaire comprenant un ou plusieurs composés choisis parmi des hydrocarbures cycliques, des hydrocarbures aliphatiques chlorés et des monoéthers, puis on distille le mélange obtenu de 0 à 60°C à la pression normale ou sous pression réduite, au moins jusqu'à ce que l'eau de réaction soit éliminée, on mélange ensuite au concentré restant une composante auxiliaire de la nature indiquée en une quantité telle que le mélange formé en contienne 5 à 50% de son poids, et on sépare par filtration la 1,3-cyclohexane-dione ayant cristallisé.

3. Procédé pour isoler la 1,3-cyclohexanedione cristallisée du produit brut formé par cyclisation catalytique en phase vapeur du 5-oxohexanoïque, procédé caractérisé en ce qu'à partir du produit brut gazeux on obtient par refroidissement entre 20 et 80°C un premier condensat, et en poursuivant le refroidissement des gaz restants entre —40 et +60°C un second condensat, on ajoute au premier condensat une quantité d'une composante auxiliaire telle que le mélange formé en contienne 5 à 50% en poids, composante auxiliaire qui est un ou plusieurs composés choisis parmi des hydrocarbures cycliques, des hydrocarbures aliphatiques chlorés et des monoéthers, et on sépare par filtration la 1,3-cyclohexane-dione ayant cristallisé, puis on ajoute au second condensat 20 à 200% de son poids d'une composante auxiliaire de la nature indiquée et on distille le mélange à 0 à 80°C à la pression normale ou sous pression réduite jusqu'à ce que l'eau de réaction soit éliminée et que la teneur du concentré restant en composante auxiliaire soit de 5 à 50% en poids, puis on sépare par filtration la 1,3-cyclohexane-dione ayant cristallisé.

4. Procédé pour isoler la 1,3-cyclohexanedione cristallisée du produit brut formé par cyclisation catalytique en phase gazeuse du 5-oxohexanoïque, procédé caractérisé en ce que l'on obtient du produit brut gazeux, par refroidissement entre 20 et 80°C, un premier condensat, puis en poursuivant le refroidissement du gaz restant entre −40 et +60°C un second condensat, on ajoute au premier condensat une quantité d'une composante auxiliaire telle que le mélange formé en contienne 5 à 50% en poids, composante qui est un ou plusieurs composés choisis parmi les hydrocarbures cycliques, des hydrocarbures aliphatiques chlorés et des monoéthers, et on sépare par filtration la 1,3-cyclohexane-dione cristalline, puis on ajoute au second condensat 10 à 100% de son poids d'une composante auxiliaire de la nature indiquée et on distille le mélange 0 à 80°C à la pression normale ou sous pression réduite, au moins jusqu'à ce que l'eau de réaction soit éliminée, au concentré restant on ajoute une quantité telle d'une composante auxiliaire de la nature indiquée que le mélange formé en contienne 5 à 50% en poids, et on sépare par filtration la 1,3-cyclohexane-dione cristalline.

5. Procédé selon la revendication 1 ou 3, caractérisé en ce que l'on prend du xylène ou de l'éther di-n-butylique comme composante auxiliaire.

6. Procédé selon la revendication 2 ou 4, caractérisé en ce que l'on prend du xylène ou du toluène comme composante auxiliaire.

7. Procédé selon la revendication 1 ou 2, caractérisé en ce que l'on mélange le produit brut gazeux, aussitôt après sa condensation, avec la composante auxiliaire, ou bien on le condense dans la composante auxiliaire placée dans un récipient.

8. Procédé selon la revendication 1 ou 2, caractérisé en ce que l'on réunit en continu à la sortie du réacteur le produit brut gazeux et la composante auxiliaire liquide, puis on condense totalement le mélange.

9. Procédé pour isoler la 1,3-cyclohexanedione cristallisée du produit brut formé par cyclisation catalytique en phase gazeuse du 5-oxohexanoïque, procédé caractérisé en ce qu'après sa condensation on chauffe le produit brut pendant une demi-heure à 20 heures entre 20 et 80°C puis, éventuellement après refroidissement, on ajoute au cristallisat brut obtenu 20 à 150% de son poids d'une composante auxiliaire qui est un ou plusieurs composés choisis parmi des hydrocarbures cycliques, des hydrocarbures aliphatiques chlorés et des monoéthers, on distille ensuite à 0 à 80°C le mélange du cristallisat brut et de la composante auxiliaire, à la pression normale ou sous pression réduite, jusqu'à ce que l'eau de réaction soit éliminée et que la teneur en composante auxiliaire du concentré restant soit de 5 à 50% en poids, et on sépare par filtration la 1,3-cyclohexane-dione ayant cristallisé.

10. Procédé pour isoler la 1,3-cyclohexanedione cristallisée du produit brut formé par cyclisation catalytique en phase vapeur du 5-oxohexanoïque, procédé caractérisé en ce que l'on chauffe le produit brut après sa condensation pendant une demi-heure à 20 heures entre 20 et 80°C, puis, éventuellement après refroidissement, on mélange le cristallisat brut obtenu avec 10 à 100% de son poids d'une composante auxiliaire comprenant un ou plusieurs composés choisis parmi des hydrocarbures cycliques, des hydrocarbures aliphatiques chlorés et des monoéthers, on distille ensuite le mélange du cristallisat brut de la composante auxiliaire entre 0 et 80°C, à la pression normale ou sous pression réduite, au moins jusqu'a ce que l'eau de réaction soit éliminée, au concentré restant on ajoute une quantité d'une composante auxiliaire de la nature indiquée telle que le mélange formé en contienne 5 à 50% en poids, et sépare par filtration la 1,3-cyclohexane-dione ayant cristallisé.

11. Procédé pour isoler la 1,3-cyclohexanedione cristallisée du produit brut formé par cyclisation catalytique en phase gazeuse du 5-oxohexanoïque, procédé caractérisé en ce que l'on obtient un premier condensat du produit brut gazeux par refroidissement entre 20 et 80°C, et en poursuivant le refroidissement des gaz restants entre −40 et +60°C un second condensat, on ajoute au premier condensat une quantité de composante auxiliaire telle que le mélange formé en contienne 5 à 50% en poids, composante qui est toujours un ou plusieurs composés choisis parmi des hydrocarbures cycliques, des hydrocarbures aliphatiques chlorés et des monoéthers, et on sépare par filtration la 1,3-cyclohexane-dione ayant cristallisé, puis on chauffe le second condensat entre 20 et 80°C pendant une demi-heure à vingt heures, on ajoute ensuite au cristallisat brut obtenu, éventuellement après refroidissement, 20 à 150% de son poids d'une composante auxiliaire de la nature indiquée et on distille le melange à 0 à 80°C à la pression normale ou sous pression réduite jusqu'à ce que l'eau de réaction soit éliminée et que la teneur du concentré restant en composante auxiliaire soit de 5 à 50% en poids, et on sépare par filtration la 1,3-cyclohexane-dione ayant cristallisé.

12. Procédé pour isoler la 1,3-cyclohexanedione cristallisée du produit brut formé par cyclisation catalytique en phase gazeuse du 5-oxohexanoïque, procédé caractérisé en ce que l'on ob-

**0 105 474**

tient un premier condensat du produit brut gazeux par refroidissement entre 20 et 80°C, et en poursuivant le refroidissement des gaz restants entre —40 et +60°C on obtient un second condensat, au premier condensat on ajoute une quantité d'une composante auxiliaire de manière que le mélange formé en contienne 5 à 50% en poids, composante qui est un ou plusieurs composés choisis parmi des hydrocarbures cycliques, des hydrocarbures aliphatiques chlorés et des monoéthers, et on sépare par filtration la 1,3-cyclohexane-dione ayant cristallisé, puis on chauffe le second condensat pendant une demi-heure à 20 heures entre 20 et 80°C et, le cas échéant après refroidissement, on mélange le cristallisat brut obtenu avec 10 à 100% de son poids d'une composante auxiliaire de la nature indiquée et on distille le mélange à 0 à 80°C, à la pression normale ou sous pression réduite, au moins jusqu'a ce que l'eau de réaction soit éliminée, puis on ajoute au concentré restant une composante auxiliaire de la nature indiquée de manière que le mélange formé en contienne 5 à 50% en poids et on sépare par filtration la 1,3-cyclohexane-dione ayant cristallisé.

13. Procédé selon la revendication 9 ou 11, caractérisé en ce que l'on emploie du xylène ou de l'éther di-n-butylique comme composante auxiliaire.

14. Procédé selon la revendication 10 ou 12, caractérisé en ce que l'on emploie du xylène ou du toluène comme composante auxiliaire.